# EUROPEAN PATENT APPLICATION

(11) **EP 2 765 180 A1**
(43) Date of publication of application: **13.08.2014**
(21) Application number: 14167659.3
(22) Date of filing: 16.09.2011
(51) Int. Cl.: C11B 1/10, B01D 17/02, C10L 1/04, C12P 7/64

(54) **Process for separation of a mixture containing a microbial oil and a microbial substance**

(30) Priority: 21.09.2010 EP 10178091; 20.12.2010 EP 10196035
(62) Divisional of application: 11757329.5
(71) Applicant: SHELL INTERNATIONAL RESEARCH MAATSCHAPPIJ B.V., 2596 HR Den Haag (NL)
(72) Inventor: Doig, Steven Daniel, Penzance, Cornwall TR20 9 SL (GB); Haan, Johannes Pieter, 1031 HW Amsterdam (NL)
(74) Representative: Matthezing, Robert Maarten

(57) **Abstract**

A process for separation of a mixture containing a microbial oil and a microbial substance into at least a first phase comprising at least part of the microbial oil and a second phase comprising at least part of the microbial substance, wherein the mixture is a fermentation broth produced by fermentation of one or more whole microorganisms capable of secreting microbial oil and wherein the microbial substance comprises one or more whole microorganisms capable of secreting microbial oil, wherein the separation is carried out by a cyclone type separation unit, wherein the process further comprises retrieving at least part of the microbial substance from the second phase and recycling at least part of the retrieved microbial substance to a fermentation process.

## Description

### TECHNICAL FIELD OF THE INVENTION

The present invention relates to a process for separation of a mixture containing a microbial oil and a microbial substance.

### BACKGROUND OF THE INVENTION

With the diminishing supply of crude mineral oil, use of renewable energy sources is becoming increasingly important for the production of chemicals and fuel products. Oil obtained from biomass, such as for example oil obtained from or via microorganisms, can be used as an alternative source to produce chemicals and fuel products. One of the advantages of using oil from such biomass is that the CO₂ balance is more favorable as compared with the conventional crude mineral oil.

Oil can for example be obtained from microorganisms by secretion of the oil by the microorganisms or by extraction of the oil after cell wall disruption of the cells of the microorganisms.

US2004/0067574 describes a process for obtaining an oil from microbial cells comprising disruption of the cell walls of the microbial cells to release the oil and separation of the oil from at least part of the cell wall debris formed. The separation of the oil from at least some of the cell wall debris is carried out by centrifugation.

US7351558 describes a process for obtaining lipids from micro-organisms comprising contacting a fermentation broth comprising the microorganisms with a base to dissolve at least a part of any proteins and subsequently increasing the temperature for lysis of the cells of the microorganism. It is described that the process can include at least partially separating the broth from the lipids. Typically this is achieved by centrifuging, that is, by passing the broth through a stacked-disc centrifuge and collecting lipids as an lipid-water emulsion phase.

CN101429467 describes a method for extracting grease and proteins from microalgae. Wet algae mud is taken as raw material and the pH value is adjusted to alkaline or slightly alkaline to perform wall breaking of microalgae cells.Dissolved microalgae slurry is filtered to separate cell residue on the one hand from a filtered liquid mixture of the grease and the proteins on the other hand. Subsequently a hydrocyclone is utilized to separate oil and water.

PACKER, M.: "Algal capture of carbon dioxide; biomass generation as a tool for greenhouse gas mitigation with reference to New Zealand energy strategy and policy", ENERGY POLICY, vol. 37, 2009, pages 3429-3437, Elsevier ISSN: 0301-4215 describes the use of hydrocyclones for algal biomass collection.

WO2010/046051 describes a process for the production of lipids.

In these processes according to the prior art, a microbial oil is separated from a microbial residue obtained after disruption of the cell walls of the microorganisms by respectively centrifugation and filtration. Separation of the microbial oil from the microbial residue by means of centrifugation has the disadvantages that energy consumption in the separation process is high and that the rotating parts of the centrifuge are prone to wear and require regular maintenance service. Especially when algae or microalgae are being processed, such rotating parts may suffer considerably from foreign matter (such as for example sand) that may be present in the microbial residue. Separation of the microbial oil from the microbial residue by means of filtration has the disadvantage that the filter gets clogged and needs regular cleaning service. Especially when algae or microalgae are being processed, a filter may get clogged by a residue that may be sticky and may contain mud (sand).

It would be an advancement in the art to provide a process for separation of a mixture comprising a microbial oil and a microbial substance, which would require less energy and/or service.

### SUMMARY OF THE INVENTION

The above has been achieved with the process according to the invention.

Accordingly, the present invention provides a process for separation of a mixture containing a microbial oil and a microbial substance into at least a first phase comprising at least part of the microbial oil and a second phase comprising at least part of the microbial substance, wherein the separation is carried out by a cyclone type separation unit.

The process according to the invention has a reduced energy consumption compared to the processes according to the prior art and requires less service.

In addition the use of a cyclone type separation unit for the separation of the microbial oil and the microbial substance allows for a continuous manner of operation of the process.

Further the process of the invention is especially advantageous when used to separate secreted microbial oil from microbial substance comprising whole microorganisms, where it is desired to avoid damage to the cell walls of the microorganisms. Because of the low local energy and low shear of the cyclone type separation unit, damage to the cell walls of the microorganisms can be kept to a minimum.

The microbial oil separated in the process according to the invention may be retrieved and subsequently hydrotreated to produce a hydrocarbon product. Such hydrocarbon product can advantageously be used in a fuel composition.

The invention therefore also provides a fuel composition containing at least part of a hydrocarbon product produced by hydrotreatment of a microbial oil, which microbial oil was obtained by separating a mixture containing a microbial oil and a microbial substance in a cyclone type separation unit into at least a first phase comprising at least part of the microbial oil and a second phase comprising at least part of the microbial substance.

### BRIEF DESCRIPTION OF THE DRAWINGS

The invention has been illustrated by the nonlimiting following figures:
Figure 1 illustrating an embodiment of the invention wherein a mixture comprising a microbial oil, water and a microbial residue obtained after lysis of microorganisms is separated.
Figure 2 illustrating an embodiment of the invention wherein a mixture comprising a microbial oil, water and whole microorganisms is separated.

### DETAILED DESCRIPTION OF THE INVENTION

The process according to the invention separates a mixture containing a microbial oil and a microbial substance. The mixture can be any mixture known in the art to contain a microbial oil and a microbial substance. Preferably the mixture contains at least a microbial oil, a microbial substance and water.

Preferably the mixture contains in the range from equal to or more than 50.0 to equal to or less than 99.8 wt% of water; in the range from equal to or more than 0.1wt% to equal to or less than 49.9 wt% of microbial substance; and in the range from equal to or more than 0.1 wt% to equal to or less than 49.9 wt% of microbial oil, based on the total weight of the mixture.

For example the mixture may contain in the range from equal to or more than 89.0 wt% to equal to or less than 99.8 wt% of water; in the range from equal to or more than 0.1 wt% to equal to or less than 1.0 wt% of microbial substance; and in the range from equal to or more than 0.1 wt% to equal to or less than 1.0 wt% of microbial oil, based on the total weight of the mixture.

This mixture may be concentrated in a concentration step before separation by a cyclone type separation unit according to the invention. Preferences for such a concentration step are described in more detail herein below. For example, the mixture may be concentrated by means of coalescence at an elevated temperature of for example equal to or more than 30°C or equal to or more than 40°C and subsequently water may be removed by decantation and/or centrifugation, whereafter the resulting concentrated mixture is used as a feed to a cyclone type separation unit.

By a microbial substance is herein understood a composition of matter from microbial origin. The microbial substance is preferably obtained from one or more microorganisms. The microbial substance can for example contain one or more whole microorganisms; and/or a microbial residue obtained after lysis of one or more microorganisms.

In one preferred embodiment the mixture containing a microbial oil and a microbial substance is a lysate mixture produced by lysis of one or more oil containing microorganisms. By lysis is herein understood disruption of the cell walls of one or more cells of the one or more microorganisms. Lysis of one or more oil containing microorganisms preferably results in release of a microbial oil, and optionally water, from the one or more microorganisms. The lysate mixture preferably contains a microbial oil, a microbial residue comprising disrupted cell walls of the cells of the one or more microorganisms, and optionally water.

In another preferred embodiment the mixture containing a microbial oil and a microbial substance is a fermentation broth produced by fermentation of one or more whole microorganisms capable of storing or secreting microbial oil. By fermentation is herein understood growing of micro-organisms under controlled circumstances. The fermentation broth preferably comprises a secreted microbial oil, one or more whole microorganisms and water. The microbial substance in the fermentation broth preferably comprises equal to or more than 50wt%, more preferably equal to or more than 70wt%, of one or more whole microorganisms, based on the total weight of microbial substance. The fermentation broth may in addition comprise less than 50wt%, more preferably less than 30wt%, of microbial residue comprising disrupted cell walls of the cells of the one or more microorganisms, based on the total weight of microbial substance. If calculation of the total weight of microbial substance is difficult the above percentages may in a preferred embodiment be calculated on the total weight of dried microbial substance.

The microorganism is preferably an organism having a diameter smaller than 1 mm, more preferably a diameter smaller than 0.6 mm and still more preferably a diameter smaller than 0.4 mm. The diameter is measured at its largest point. Most preferably the microorganism comprises a diameter in the range from 0.5 to 200 micrometer, even more preferably in the range from 1 to 100 micrometer.

A wide variety of microorganisms can be used in the microbial substance.

The one or more microorganisms can for example include autotrophic and/or heterotrophic microorganisms. By an autotrophic microorganism is herein understood a microorganisms that obtains carbon by fixing carbon dioxide. By an heterotrophic microorganism is herein understood a microorganism that obtains carbon from organic carbon sources, such as for example carbohydrates. The energy metabolism of the microorganism can for example be based on phototrophy (that is where the microorganism uses light as its source of energy) or it can be based on chemotrophy (that is where the microorganism uses organic substances as its source of energy). The one or more microorganisms can include aerobic and/or anaerobic microorganisms.

The microorganisms are preferably unicellular. The microorganisms may be present as separated unicellular microorganisms or as a colony of unicellular microorganisms. The one or more microorganisms can include so-called wild-type microorganisms and/or genetically modified microorganisms.

The one or more microorganisms can include microorganisms that merely store microbial oil and/or microorganisms that secret microbial oil.

Micro-organisms that store and/or secret microbial oil are sometimes also referred to as oleaginous micro-organisms. The microbial substance preferably comprises one or more oleaginous microorganisms. The oleaginous microorganism preferably is a microorganism able to store oil in its interior in an amount of equal to or more than 10wt%, more preferably equal to or more than 15wt%, still more preferably equal to or more than 20wt% and most preferably equal to or more than 30wt% of its dry biomass.

Preferably the microbial substance comprises one or more microorganisms chosen from the group consisting of bacteria, fungi, yeasts, algae and mixtures thereof.

Examples of bacteria include Rhodococcus, Mycobacteria, Vibrio, Escherichia coli, Bacillus, Brevibacterium, Corynebacterium, Flavobacterium, Klebsiella, Micrococcus, Mycoplana, Paracoccus, Pseudomonas, Acetobacter and/or other bacteria and/or genetically modified strains thereof and/or mixtures thereof.

In one preferred embodiment the one or more microorganisms include fungi. Examples of such fungi include fungi of the genera Saprolegnia,Phytophthora, Mucor, Rhizopus, Absidia, Mortierella, Cunninghamella, Taphrina, Monascus, Nectria, Gibberella, Chaetomium, Neurospora, Geotrichum, Monilia,Trichoderma, Aspergillus, Penicillium, Paecilomyces, Gliocladium, Sporotrichum, Microsporum, Trichophyton, Cladosporium, Syncephalastrum, Phycomyces and Eupenicillium and/or genetically modified strains thereof and/or mixtures thereof.

In another preferred embodiment the one or more microorganisms include yeasts. Yeasts are eukaryotic micro-organisms classified in the kingdom Fungi.

A wide range of yeasts can be used, including for example yeasts of the genera Endomyces, Shizosaccharomyces, Pichia, Hansenula, Debaryomyces, Saccharomyces , Saccharomycopsis, Rhodotorula, Sporobolomyces, Cryptococcus, Candida,and Brettanomyces, Lipomyces, Endomycopsis, Rhodosporidium, Yarrowia and/or genetically modified strains thereof and/or mixtures thereof.

In still another preferred embodiment the one or more microorganisms include algae, most preferably microalgae. By microalgae are herein understood unicellular microorganisms capable of photosynthesis. Such microalgae include for example diatomic algae, green algae and cyanobacteria, but exclude seaweed. More preferably the microorganisms are eukaryotic microalgae.

The algae may be sweet water algae or salt water algae. Preferably, the microalgae used in the process of the present invention are marine microalgae.

A wide range of microalgae can be used, including for example Botryococcus braunii, Chlorella, Dunaliella tertiolecta, Gracilaria, Pleurochrysis carterae and/or genetically modified strains thereof and/or mixtures thereof. The algae may be sweet water algae or salt water algae. Preferably, the microalgae used in the process of the present invention are marine microalgae. Suitable marine microalgae can include members from various divisions of algae, including for example diatoms, pyrrophyta, nitszchia, porphyridia, ochrophyta, chlorophyta, euglenophyta, dinoflagellata, chrysophyta, crypthecodinia, phaeophyta, rhodophyta and cyanobacteria. Preferably, the marine microalgae are members from the diatoms or ochrophyta division, more preferably from the raphid, araphid, and centric diatom family.

The one or more microorganisms can be produced by cultivation and/or fermentation in any manner known by the skilled in the art to be suitable for this purpose. For example the cultivation and/or fermentation of the one or more microorganisms can be carried out under open culture conditions or closed culture conditions.

If the microbial substance contains oleaginous yeasts, such oleaginous yeasts are preferably fermented under closed-culture conditions, preferably in closed bioreactors. The yeasts can be fermented at a wide range of fermentation conditions. Preferably the yeasts are fermented at a pH condition in the range from 2.5 to 7, more preferably in the range from 3 to 5. The temperature during fermentation preferably lies in the range from 20°C to 40°C, more preferably in the range from 25°C to 35°C. During fermentation, the bioreactor is preferably aerated to a saturation level in the range of 1 to 50%, more preferably 10 to 30% of the complete saturation of the water with air. The oleaginous yeasts can be fermented in any culture medium known by those skilled in the art to be suitable for this purpose. Preferably the culture medium comprises a carbon source. A preferred carbon source are sugars. These sugars can include monosaccharides, disaccharides, trisaccharides and polysaccharides. Examples of sugars that can be comprised in the culture medium include for example fructose, galactose, glucose, sucrose, xylose, maltose, sacharose, lactose, dextrose and corn syrup. In an especially preferred embodiment sugar cane, more preferably Brazilian sugar cane is used as a carbon source. The carbon source is preferably present in a concentration of from 2 to 35 wt%, more preferably in a concentration of from 5 to 25 wt%. The oleaginous yeasts are preferably present in a concentration of 1 to 20 wt%, more preferably in a concentration of 5 to 15 wt%.

The oleaginous yeasts can be grown in various manners, including in a continuous mode, in a continuous mode with a recycle of yeast cells, batch-wise, or in a fed-batch mode (wherein growth of the oleaginous yeasts is controlled by feeding a controlled amount of nutrient substrate to the culture medium).

If the microbial substance contains microalgae, such microalgae can be cultivated under open or closed culture conditions. Closed culture conditions may include for example closed photo-bioreactors (that is a closed bioreactor that allows the microalgae to have access to light). Open-culture conditions may include for example open sea or ponds. The microalgae can for example be cultivated in freshwater, sweet water, saline water or moist earth.

By a microbial oil is herein understood an oil from microbial origin. The microbial oil may for example be obtained via secretion by one or more oil-secreting microorganisms and/or via extraction from one or more oil-storing microorganisms.

The microbial oil preferably contains one or more lipids. Preferably the lipids comprise naturally occurring compounds that are essentially hydrophobic in nature and contain long-chain aliphatic hydrocarbons. The one or more lipids preferably comprise monoglycerides, diglycerides and/or triglycerides (which are mono- di and tri-esters of glycerol and fatty acids); phospholipids (which are esters of glycerol and phosphate group-substituted fatty acids); and glycolipids (which are esters of fatty acids and sugars).

The fatty acid moiety in the one or more lipids can comprise saturated fatty acids and/or unsaturated fatty acids containing one, two, three or more double bonds.

Preferably the one or more fatty acid moiety(ies) in the one or more lipids comprises equal to or more than 4 carbon atoms, more preferably equal to or more than 8 carbon atoms, still more preferably equal to or more than 10 carbon atoms, yet still more preferably equal to or more than 12 carbon atoms and most preferably equal to or more than 14 carbon atoms. The fatty acid moiety in the one or more lipids further preferably comprises equal to or less than 30 carbon atoms, more preferably equal to or less than 26 carbon atoms, still more preferably equal to or less than 25 carbon atoms, yet still more preferably equal to or less than 23 carbon atoms and most preferably equal to or less than 20 carbon atoms.

The microbial oil can further contain a wide range additional compounds. For example, the microbial oil can further contain (saturated or unsaturated) free fatty acids and/or esters thereof; fatty alcohols and/or fatty amines; carotenoids; terpenes; styrols; tocopherols; and/or proteins.

In addition to the microbial oil and the microbial substance, the mixture to be separated in the process of the invention may contain one or more other components. Examples include water and optionally sand.

In a preferred embodiment the mixture to be separated in the process according to the invention comprises at least a microbial oil, a microbial substance and water. More preferably the mixture consists essentially of microbial oil, microbial substance and water. For example the mixture to be separated may consist of an aqueous suspension of microbial oil and microbial substance.

The separation according to the invention is carried out by a cyclone type separation unit. The cyclone type separation unit may comprise one, two or more cyclone type separators. In one preferred embodiment the cyclone type separation unit comprises one or two cyclone type separators. In another preferred embodiment the cyclone type separation unit comprises equal to or more than 3 cyclone type separators, more preferably equal to or more than 5 cyclone type separators, and equal to or less than 25 cyclone type separators, more preferably equal to or less than 20 cyclone type separators.

If the cyclone type separation unit comprises two or more cyclone type separator, the two or more cyclone type separators may be arranged in parallel or in series. Preferably the two or more cyclone type separators are arranged in series. In a preferred embodiment where the cyclone type separation unit comprises two or more cyclone type separators connected in series, the top outlet of a preceding cyclone type separator is preferably coupled directly to the inlet of a subsequent cyclone type separator. In such a series of two or more cyclone type separators preferably one or more preceding cyclone type separator(s) may have a larger diameter than one or more subsequent cyclone type separator(s). Such an arrangement advantageously allows for declining volumes to be processed through the cyclone type separators and saves on building costs.

A cyclone type separator preferably does not comprise a rotating body. Instead a cyclone type separator preferably causes the feed to make a rotating movement, by feeding such feed to the separation unit in a tangential manner.

Preferably the cyclone type separator comprises a cylindrically shaped top section and a conically shaped bottom section, where the central axis of the cylindrically shaped top section and the conically shaped bottom section are essentially aligned. The cylindrically shaped top section preferably comprises a tubular housing with a tangentially arranged inlet and a top cover protruded by a top outlet located essentially along the central axis of the tubular housing. The conically shaped bottom section is preferably fluidly connected to the cylindrically shaped top section and preferably comprises a conically shaped housing comprising a bottom outlet located essentially along the central axis of the conically shaped housing.

Preferably the cyclone type separation unit comprises one or more hydro-cyclone(s). By a hydrocyclone is herein understood a cyclone type separator designed for the separation of mixtures containing water into at least a first phase and at least a second phase, wherein at least the first phase or the second phase contains water.

The cyclone type separator may have any dimensions known to the person skilled in the art to be suitable for separating a mixture containing solids and liquids. In a preferred embodiment the ratio of the diameter of the tubular housing (Dₜₕ) in the cylindrically shaped top section to the diameter of the bottom outlet (D_{bo}) in the conically shaped bottom section lies in the range from 30:1 to 1.5:1, more preferably in the range from 15:1 to 2:1. Further the angle α between the central axis of the conically shaped bottom section and the inner wall of the conically shaped housing preferably lies in the range from 0.5° to 60°, more preferably in the range from 1° to 45° and still more preferably in the range from 5° to 30°.

In a further preferred embodiment the one or more cyclone type separators comprise a dipleg fluidly connected to the bottom outlet of the cyclone type separator. Preferably the dipleg(s) are sealed to prevent an upward flow into the cyclone type separator, for example by submerging the bottom of the dipleg(s) in an aqueous suspension of microbial substance.

The one or more cyclone type separators can be manufactured from any type of material known by the skilled person in the art to be suitable for separation of a liquid and a solid. Examples of materials that can be used in the manufacture of the cyclone type separator include metal (for example steel or stainless steel); ceramics or plastics such as polyurethane or polypropylene. In a preferred embodiment the cyclone type separation unit comprises one or more cyclone type separators that are manufactured from an abrasion resistant material or are lined with an abrasion resistant material. This is especially advantageous where the mixture comprising microbial oil and microbial substance comprises microalgae and/or sand. An advantage of using a cyclone type separator comprising such an abrasion resistant material is that the cyclone type separator will be less prone to wear.

More preferably the cyclone type separation unit comprises one or more cyclone type separator(s) manufactured from a pressure resistant material lined with an abrasion resistant material. Most preferably the cyclone type separation unit comprises one or more cyclone type separator(s) manufactured from a metal such as for example stainless steel lined with a plastic such as for example polyurethane.

In the process of the invention the mixture comprising a microbial oil and a microbial substance is separated into at least a first phase comprising at least part of the microbial oil and a second phase comprising at least part of the microbial substance. Preferably the first phase comprising at least part of the microbial oil is the light phase and preferably the second phase comprising at least part of the microbial substance is the heavy phase.

The light phase preferably leaves the cyclone type separation unit via the top outlet of the (last) cyclone type separator and the heavy phase preferably leaves the cyclone type separation unit via the bottom outlet(s) of the one or more cyclone type separators.

The first phase preferably contains microbial oil and optionally a small percentage of water and/or a small percentage of microbial substance. The second phase preferably contains microbial substance and optionally water and/or a small percentage of microbial oil.

Preferably the first phase contains equal to or more than 50wt% of microbial oil, more preferably equal to or more than 60wt% and still more preferably equal to or more than 70wt% of microbial oil, based on the total weight of first phase. Even still more preferably the first phase contains equal to to or more than 80 wt% of microbial oil, most preferably equal to or more than 85wt% of microbial oil, based on the total weight of first phase. The first phase may consist essentially of microbial oil (for example 100wt%), but can contain equal to or less than 98 or 99 wt%, or equal to or less than 95wt% or equal to or less than 90wt% of microbial oil, based on the total weight of first phase.

If the first phase contains water, the first phase preferably contains equal to or less than 50wt% of water, more preferably equal or less than 40wt% and still more preferably equal or less than 30wt% of water, based on the total weight of first phase. Even still more preferably the first phase contains equal to or less than 20 wt% of water, most preferably equal to or less than 15wt% of water. The first phase most preferably contains essentially no water( for example 0wt%), but can contain equal or more than 1 or 2wt%, or equal or more than 5wt% of water, based on the total weight of first phase.

If the first phase contains microbial substance, the first phase preferably contains equal to or less than 50wt% of microbial substance, more preferably equal or less than 40wt% and still more preferably equal or less than 30wt% of microbial substance, based on the total weight of first phase. Even still more preferably the first phase contains equal to or less than 20 wt% of microbial substance, most preferably equal to or less than 15wt% of microbial substance. The first phase preferably contains essentially no microbial substance (for example 0wt%), but can contain equal or more than 1 or 2wt%, or equal or more than 5wt% of microbial substance, based on the total weight of first phase.

Preferably the second phase contains equal to or more than 90wt% of microbial residue and/or water, more preferably equal or more than 95wt% of microbial residue and/or water, based on the total weight of second phase. Still more preferably the second phase contains equal to or more than 99wt% of microbial residue and/or water, most preferably equal to or more than 99.5wt% of microbial residue and optionally water, based on the total weight of second phase. The second phase preferably consists essentially of microbial residue and/or water (for example 100wt%), but can contain equal or less than 99.9 wt% of microbial residue and/or water, based on the total weight of second phase.

If the second phase contains microbial oil, the second phase preferably contains equal to or less than 10wt% of microbial oil, more preferably equal or less than 5wt% and still more preferably equal or less than 1wt% of microbial oil, based on the total weight of second phase. Even still more preferably the second phase contains equal to or less than 0.5 wt% of microbial oil. The second phase preferably contains essentially no microbial oil( for example 0wt%), but can contain equal or more than 0.1wt%, of microbial oil, based on the total weight of second phase.

In an especially preferred embodiment, the separation is carried out such that the first phase contains essentially only microbial oil and essentially no water and the second phase contains the microbial substance and any optional water.

In many cases it may be advantageous to retrieve at least part of the microbial substance from the second phase. Retrieving the microbial substance from the second phase can be achieved by any method known by the skilled person in the art to be suitable for this purpose. For example microbial substance may be retrieved by means of solid/liquid separation and/or sedimentation.

In one preferred embodiment, where the microbial substance comprises one or more whole microorganisms capable of storing or secreting microbial oil the process preferably comprises retrieving at least part of the microbial substance from the second phase and recycling of at least part of the retrieved microbial substance to a fermentation process.

In another preferred embodiment, where the microbial substance comprises disrupted cell walls of the cells of one or more microorganisms the process preferably comprises retrieving at least part of the microbial substance from the second phase and use of the retrieved microbial substance as animal feed (including fish feed)or as a feedstock to produce methane. The methane can advantageously be used as a burning fuel. In some cases it may further be advantageous to burn the retrieved microbial residue as such.

In a preferred embodiment of the process at least part of the microbial oil may advantageously be retrieved from the first phase. If necessary, retrieving the microbial oil from the first phase can be achieved by any method known by the skilled person in the art to be suitable for this purpose. Suitable methods may for example include solvent distillation, extraction, centrifugation and separation by means of a cyclone type separation unit. In a preferred embodiment the first phase contains essentially 100wt% of microbial oil and the retrieval of the microbial oil comprises merely the transport from one location, for example the top outlet of the last cyclone type separator, to another location, for example a storage vessel or the inlet of a next processing unit.

The retrieved microbial oil can advantageously be used for the production of chemicals and fuel products. In a preferred embodiment the retrieved microbial oil is hydrotreated in a further step to produce a hydrocarbon product. Such hydrotreatment can for example include hydrogenation and/or hydrodeoxygenation and/or hydroisomerization.

In another preferred embodiment the retrieved microbial oil is enzymatically converted in a further step to produce a hydrocarbon product.

In a further preferred embodiment the hydrocarbon product produced is blended with one or more other components to produce a fuel composition.

The process according to the invention can be carried out in a batchwise, semi-batchwise or continuous manner. Preferably the process according to the invention is carried out in a continuous manner. The use of the cyclone type separation unit advantageously allows for such a continuous operation.

As indicated above, in one preferred embodiment of the invention the mixture containing at least a microbial oil and a microbial substance is a lysate mixture produced by lysis of one or more oil containing microorganisms.

The invention therefore also provides a process for producing an oil from a feed containing one or more microorganisms and optionally water, comprising
a) lysis of the one or more microorganisms to produce a lysate mixture comprising a microbial oil, a microbial residue comprising disrupted cell walls of the cells of the one or more microorganisms, and optionally water;
b) separation of the lysate mixture into at least a first phase comprising at least part of the microbial oil and a second phase comprising at least part of the microbial residue and optionally water;
c) retrieval of the microbial oil from the first phase; wherein the separation in step b) is carried out by a cyclone type separation unit.

Preferences for such a process, especially for the one or more microorganisms, the microbial oil and the cyclone type separation unit, are as described above. In an especially preferred embodiment the feed contains one or more oleaginous microorganisms.

In a preferred embodiment the feed to such a process comprises an aqueous slurry of the one or more microorganisms in water.

In another preferred embodiment step a) comprises the release of water contained in the cells of the one or more microorganisms during lysis.

In a preferred embodiment, the feed containing one or more microorganisms is subjected to a concentration step after cultivation and/or fermentation and before use in step a) or a concentration step is carried out in between step a) and b) of the process according to the invention. Such a concentration step has the advantage that the volume of material to be processed is reduced and/or less energy is needed to process large amounts of solvent (for example water) in or after step a). The feed may for example be concentrated with help of centrifugation, filtration, flotation, coalescence, sedimentation and/or flocculation and/or decantation and/or by making use of a cyclone type separation unit.

It may be preferred to carry out the concentration step in the presence of a salt and/or additive, and/or at a temperature of equal to or more than 30°C or equal to or more than 40°C to allow for a better concentration.

The feed containing one or more microorganisms that is fed into step a) preferably has a dry matter content in the range from 0.1wt% to 50wt%, more preferably in the range from 0.5wt% to 30wt%, most preferably in the range from 1wt% to 20wt%.

In step a) the one or more microorganisms are subjected to lysis. Lysis may be achieved by any manner known by the person skilled in the art to be suitable for this purpose. For example lysis may be achieved with the help of enzymatic, physical, chemical, osmotic or mechanical methods or a mixture thereof.

An example of a physical method is the heating and/or drying of the microorganisms at an elevated temperature suitable to rupture the cell walls of one or more cells of the microorganisms. In this case the microorganisms are preferably heated to a temperature of equal or more than 50°C, more preferably of equal or more than 75°C, still more preferably of equal or more than 100°C and most preferably of equal or more than 120°C. Preferred physical methods to achieve lysis include for example boiling or steam treatment of the feed comprising one or more microorganisms.

An example of an osmotic method is the treatment of the one or more microorganisms in a hypotonic environment where the surrounding fluid has a lower salt concentration than the interior of the cells of the one or more microorganisms.

Examples of chemical methods include the treatment of the feed comprising one or more microorganisms with a base or acid or a surfactant or detergent. By a base is understood any compound whose pKa is greater than that of water. In a preferred embodiment a base is used selected from the group consisting of hydroxides, carbonates and bicarbonates of lithium, sodium, potassium, calcium and magnesium. By an acid is understood any compound whose pKa is smaller than that of water. In a preferred embodiment an acid is used selected from the group consisting of sulphuric acids, phosphoric acids, hydrochloric acid, formic acid, acetic acid, citric acid.

An example of an enzymatic method includes treatment of the feed comprising one or more microorganisms with cell wall degrading enzymes. Examples of cell wall degrading enzymes include proteases, cellulases, hemicellulases, chitinases and /or pectinases.

An example of a mechanical method is the treatment of the feed comprising one or more microorganisms with ultrasound. In such a case preferably a frequency in the range from 20-50 KHz is applied. Another example of a mechanical method is the treatment of the feed comprising one or more microorganisms with a high-shear device (also sometimes referred to as a homogenisation), such as for example a rotor-stator disruptor or valve type processor. Valve-type processors wherein one or more cells of one or more microorganisms are disrupted by forcing the feed through a narrow valve under high pressure (preferably a pressure in the range from 100 to 1000 bar, more preferably 200 to 600 bar) are preferred. Other examples of homogenisation include treatment with a ball-mill or bead-mill (e.g. including sand and/or glass beads).

In step b) the lysate mixture is separated according to the separation process of the invention to produce a first phase containing at least part of the microbial oil and a second phase containing at least part of the microbial residue by means of the cyclone type separation unit.

If the lysate mixture contained water, the first phase may contain microbial oil and water and the second phase may contain microbial residue and water. In a preferred embodiment, however, the separation is carried out such that the first phase contains microbial oil and essentially no water and the second phase contains the microbial residue and water and essentially no microbial oil.

Further preferences for the separation step b) and/or retrieval step c) are as described above for the separation process according to the invention.

In a further embodiment an extraction solvent is added to the lysate mixture produced in step a) before separation of said lysate mixture in step b); and wherein in step b) the lysate mixture is separated into a first phase containing at least part of the microbial oil and at least part of the extraction solvent and a second phase containing at least part of the microbial residue and optionally water. Use of such an extraction solvent advantageously improves the extent of separation between the microbial oil and the microbial residue and optionally water. Examples of extraction solvents that can be used include C1-C10 alkyl esters, such as ethyl acetate or butyl acetate; toluene; C4-C10 alcohols, such as for example pentanol, hexanol, ethyl hexanol; C3-C8 alkanes, such as for example hexane or heptanes.

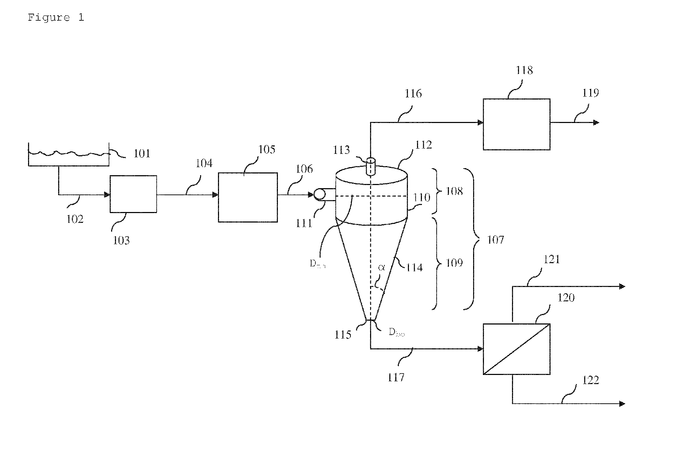

One embodiment of the invention, wherein a lysate mixture comprising a microbial oil, water and microbial residue obtained after lysis of microorganisms is separated, is illustrated in figure 1.

In the flowsheet of figure 1 microalgae are cultivated in an open pond (101). The microalgae are harvested from the open pond (101) and a feed stream comprising the microalgae and water is fed via line (102) into a concentration unit (103). In the concentration unit (103) the feed stream is concentrated to produce an aqueous concentrated feed stream (104) comprising 15 wt% of algae on a dry basis. The concentrated feed stream (104) is forwarded into a lysis unit (105), where the cell walls of the microalgae are disrupted, to produce a lysate (106) comprising water, algal oil and cell wall residue. The lysate (106) is forwarded into a hydrocyclone (107). The hydrocyclone (107) comprises a cylindrically shaped top section (108) and a conically shaped bottom section (109), where the central axis of the cylindrically shaped top section (108) and the conically shaped bottom section (109) are essentially aligned. The cylindrically shaped top section (108) comprises a tubular housing (110) with a tangentially arranged inlet (111) and a top cover (112) protruded by a top outlet (113) located essentially along the central axis of the tubular housing (110). The conically shaped bottom section (109) comprises a conically shaped housing (114) comprising a bottom outlet (115) located essentially along the central axis of the conically shaped housing (114). The central axis of the tubular housing (110) and the central axis of the conically shaped housing (114) essentially coincide.

The lysate (106) enters the hydrocyclone (107) via the tangentially arranged inlet (111). In the hydrocyclone (107) the lysate is separated into a stream (116) containing an oily phase that leaves the hydrocyclone (107) via the top outlet (113) and a stream (117) comprising an aqueous phase that contains a microbial residue comprising disrupted cell walls of the cells of the microalgae, which stream (117) leaves the hydrocyclone (107) via bottom outlet (115).

The stream (116) containing the oily phase is forwarded to a hydrotreatment unit (118), where the oily phase is hydro-deoxygenated, hydrogenated and/or hydroisomerized. In the hydrotreatment unit (118) a stream (119) is produced comprising hydrocarbons. The stream (119) comprising hydrocarbons may be used in the preparation of a fuel.

Stream (117) comprising an aqueous phase that contains the microbial residue comprising disrupted cell walls of the cells of the microalgae is forwarded to a solid liquid separator (120) where water and residue are separated to produce a stream of waste water (121) and a stream of microbial residue (122).

In another preferred embodiment the mixture containing at least a microbial oil and a microbial substance is a fermentation broth produced by fermentation of one or more microorganisms capable of secreting microbial oil. In such an embodiment the microbial oil comprises the microbial oil secreted by the one or more microorganisms and the microbial substance comprises the one or more, preferably whole, microorganisms.

The invention therefore also provides a process for producing an oil from a feed containing one or more whole microorganisms and optionally water, comprising
i) fermentation of the one or more microorganisms in a bio reactor under conditions suitable for secretion of microbial oil by the one or more microorganisms to produce a fermentation broth comprising secreted microbial oil, one or more whole microorganisms, and optionally water;
ii) separation of the fermentation broth into at least a first phase containing at least part of the secreted microbial oil and a second phase containing at least part of the one or more whole micro-organisms and optionally water;
iii) retrieval of the secreted microbial oil from the first phase;
wherein the separation in step ii) is carried out by a cyclone type separation unit.

Preferences for such a process, especially for the one or more microorganisms, the microbial oil and the cyclone type separation unit, are as described above.

In a preferred embodiment the feed to such a process comprises an aqueous slurry of the one or more microorganisms in water. In a further preferred embodiment, the feed containing one or more microorganisms is subjected to a concentration step before or after fermentation in step i) and before separation in step ii) of the process. Such a concentration step has the advantage that the volume of material to be processed in step i) and/or ii) is reduced and less energy is needed to process large amounts of for example water in step i) and/or ii). The feed may for example be concentrated with help of centrifugation, filtration, flotation, coalescence, sedimentation and/or flocculation and/or decantation and/or by making use of a cyclone type separation unit.

It may be preferred to carry out the concentration step in the presence of a salt and/or additive, and/or at a temperature of equal to or more than 30°C or equal to or more than 40°C to allow for a better concentration.

Further preferences for the separation step ii) and/or retrieval step iii) are as described above for the separation process according to the invention.

In a preferred embodiment the process comprises a further step iv) comprising retrieval of the one or more whole microorganisms from the second phase.

In a further preferred embodiment the one or more whole microorganisms are retrieved from the second phase in such a step iv) and subsequently forwarded to a fermentation process for re-use. More preferably the retrieved one or more whole microorganisms are recycled to fermentation step i)in the same process.

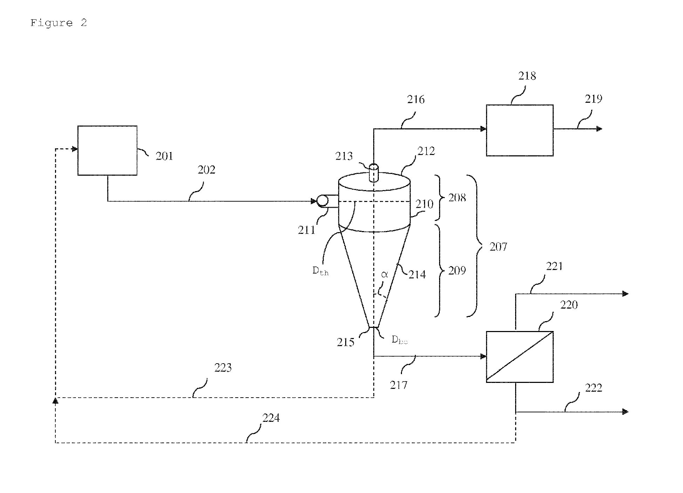

Figure 2 illustrates an embodiment of the invention wherein a mixture comprising a microbial oil, water and whole microorganisms is separated.

In Figure 2 yeast cells are fermented in a closed bio-reactor (201). During the fermentation a microbial oil is secreted by the yeast cells. A stream of fermentation broth (202) containing whole yeast cells, water and microbial oil is withdrawn from the closed bioreactor (201) and forwarded into a hydrocyclone (207). The hydrocyclone (207) comprises a cylindrically shaped top section (208) and a conically shaped bottom section (209), where the central axis of the cylindrically shaped top section (208) and the conically shaped bottom section (209) are essentially aligned. The cylindrically shaped top section (208) comprises a tubular housing (210) with a tangentially arranged inlet (211) and a top cover (212) protruded by a top outlet (213) located essentially along the central axis of the tubular housing (210). The conically shaped bottom section (209) comprises a conically shaped housing (214) comprising a bottom outlet (215) located essentially along the central axis of the conically shaped housing (214). The central axis of the tubular housing (210) and the central axis of the conically shaped housing (214) essentially coincide.

The fermentation broth (202) enters the hydrocyclone (207) via the tangentially arranged inlet (211). In the hydrocyclone (207) the fermentation broth is separated into a stream (216) containing an oily phase comprising microbial oil that leaves the hydrocyclone (207) via the top outlet (213) and a stream (217) comprising an aqueous phase that contains the whole yeast cells, which stream (217) leaves the hydrocyclone (207) via bottom outlet (215).

In a special embodiment at least part of the aqueous phase containing whole yeast cells leaving the hydrocyclone (207) via bottom outlet (215) may be recycled back to the closed bio-reactor (201) via a first recycle stream (223).

The stream (216) containing the oily phase is forwarded to a hydrotreatment unit (218), where the oily phase is hydro-deoxygenated, hydrogenated and/or hydroisomerized. In the hydrotreatment unit (218) a stream (219) is produced comprising hydrocarbons. The stream (219) comprising hydrocarbons may be used in the preparation of a fuel.

Stream (217) comprising an aqueous phase that contains the whole yeast cells is forwarded to a solid liquid separator (220) where water and whole yeast cells are separated to produce a stream of waste water (221) and a stream of whole yeast cells (222). In a further special embodiment at least part of the stream of whole yeast cells (222) may be recycled back to the closed bio-reactor (201) via a second recycle stream (224).

## Claims

1. A process for separation of a mixture containing a microbial oil and a microbial substance into at least a first phase comprising at least part of the microbial oil and a second phase comprising at least part of the microbial substance, wherein the mixture is a fermentation broth produced by fermentation of one or more whole microorganisms capable of secreting microbial oil and wherein the microbial substance comprises one or more whole microorganisms capable of secreting microbial oil, wherein the separation is carried out by a cyclone type separation unit, wherein the process further comprises retrieving at least part of the microbial substance from the second phase and recycling at least part of the retrieved microbial substance to a fermentation process.

2. The process of claim 1, wherein the mixture comprises a microbial oil, a microbial substance and water.

3. The process of anyone of claims 1 to 2, wherein the microbial substance comprises one or more autotrophic and/or heterotrophic microorganisms.

4. The process of anyone of claims 1 to 3, wherein the microbial substance comprises one or more microorganisms chosen from the group consisting of bacteria, fungi, yeasts, algae or mixtures thereof.

5. The process of anyone of claims 1 to 4, wherein the microbial substance comprises one or more marine microalgae.

6. The process of anyone of claims 1 to 4, wherein the microbial substance comprises one or more yeasts.

7. The process of anyone of claims 1 to 6, wherein the process further comprises retrieving at least part of the microbial oil from the first phase.

8. The process of claim 7, wherein the process further comprises hydrotreating of the retrieved microbial oil to produce a hydrocarbon product.

9. The process of claim 8, wherein the process further comprises blending the hydrocarbon product with one or more other components to produce a fuel composition.

10. The process of anyone of claims 1 to 4 and 7 - 9 wherein the microbial substance comprises one or more bacteria.
